# EUROPEAN PATENT APPLICATION

(11) **EP 2 952 508 A1**
(43) Date of publication of application: **09.12.2015**
(21) Application number: 14171184.6
(22) Date of filing: 04.06.2014
(51) Int. Cl.: C07D 251/70

(54) **Method for modifying a triazine compound**

(71) Applicant: Borealis Agrolinz Melamine GmbH, 4021 Linz (AT)
(72) Inventor: Dicke, René, Dr., 4060 Leonding (AT)
(74) Representative: Maikowski & Ninnemann

(57) **Abstract**

The present invention relates to a method for modifying a triazine compound
comprising the steps of
a) Providing at least one triazine compound of the general formulae (I) 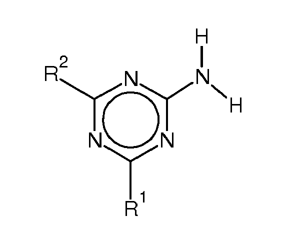 wherein
- R¹ and R² mean independently from each other Q¹ or a moiety of the formula R³-N-R⁴ or R⁵-N-R⁶ bound with its central nitrogen atom to the triazine ring of the structure of formula (I), whereat
- Q¹ means H, hydroxyl, a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl, wherein in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or -OC(O)O-, and/or can be functionalized by one or multiple hydroxyl groups and/or mercapto groups,

- R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and

b) Reacting said aminotriazine of the general formulae (I) with at least one ammonium salt of the general formulae (II)

[(R⁷R⁸H₂N⁺]ₙXⁿ⁻

wherein R⁷ and R⁸ linear or branched C₁-C₂₀-alkyl, C₅-C₂₀-cycloalkyl, or C₁-C₂₀-alkylsubstituted C₅-C₂₀-aryl or cyclic amines, or wherein R⁷ and R⁸ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O- and/or can be functionalized by one or multiple hydroxyl groups and/or mercapto groups, and wherein R⁷ and R⁸ can be the same or different;
wherein Xⁿ⁻ is an anion of an organic or inorganic acid, and
wherein n ≥1, in particular 1, 2 or 3, and
c) wherein the reaction takes place in a salt melt of the at least one ammonium salt of the general formuale (II).

## Description

The invention relates to a method for modifying a triazine compound according to the preamble of claim 1.

### Description

Alkylated triazines such as alkylated melamines are well known and have a broad field of application. For instance, methyl melamines as only one example of alkylated melamines are used as a pharmaceutical compound in cancer therapy, as biocides or insecticides (Calgon 1967, Cassella 1965), as a resin component (Nissan 1995, BASF 1964), as hardener or linking agent (Nissan 1994, Allied Chemistry 1964, Monsanto 1957, Cytec 2003) as an ingredient of stabilizing mixtures for plastics (Nissan 1995, American Cyanamid 1968), in the production of polyimides (Kanebo 1982) as well as in flame retardants, textiles and ion exchange resins.

Due to the broad application spectrum, there is a desire to provide a convenient method for obtaining alkylated triazines such as alkylated melamines with high yields, low impurity content and tailor-made degree of substitution, such as for instance adjusting the ratio of N,N-dialkylated melamine (DMM) and N,N,N',N'-tetraalkylated melamine (TMM) .

Different approaches of synthesizing alkylated triazines such as methyl melamine derivatives have been described in the past. Only exemplarily the following methods are mentioned:
- Conversion of melamine with methyl amine salts (IG Farben, Nissan),
- catalytic hydrogenation of methyl etherified melamine formaldehyde resins using suitable catalysts such as Raney nickel (Casella),
- reductive alkylation of melamine by converting melamine with aldehydes / ketones in the presence of a suitable catalyst and hydrogen (Nissan),
- alkylation of melamine with alkyl halogenids using a basic catalyst (Nissan 1995),
- catalytic alkylation of melamine with olefins (Nissan 1994) or
- conversion of cyan guanidine (dicyandiamide) with dimethyl sulphate as methylating agent, methyl amines or methyl cyanamides (American Cyanamid 1953).

These known synthesis reactions have the disadvantage of by products or the use of toxic educts, such as cyanuric chloride.

A further approach for obtaining alkylated triazines, such as methylated melamines, is the transamination of the triazine with amines, in particular with primary or secondary amines. The transamination reaction is usually carried out at high temperatures of about 200°. However, the amines used for transamination are usually in a gaseous form under these temperature conditions and thus the reaction has to be carried out under high pressure, such as a pressure autoclave, making the overall reaction rather cost intensive and thus not practicable for large scale industrial application.

It was therefore an object of the present invention to provide a method which allows for a specific synthesis of substituted triazines, in which the disadvantages of the prior art are overcome.

This object is being solved with the method comprising the features of claim 1.

Accordingly, a method for obtaining or modifying a triazine compound, in particular by transamination, is provided which comprises the steps of
a) providing at least one triazine compound of the general formulae (I) 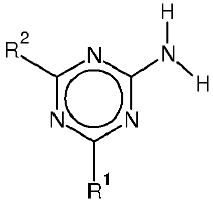 wherein
   - R¹ and R² mean independently from each other Q¹ or a moiety of the formula R³-N-R⁴ or R⁵-N-R⁶ bound with its central nitrogen atom to the triazine ring of the structure of formula (I), whereat
      - Q¹ means H, hydroxyl, a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl, wherein in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or -OC(O)O-, and/or can be functionalized by one or multiple hydroxyl groups and/or mercapto groups,
   - R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and
b) reacting said aminotriazine of the general formulae (I) with at least one ammonium salt of the general formulae (II)

   [(R⁷R⁸H₂)N⁺]ₙxⁿ⁻

   wherein R⁷, and R⁸, are linear or branched C₁-C₂₀-alkyl, C₅-C₂₀-cycloalkyl, or C₁-C₂₀-alkylsubstituted C₅-C₂₀-aryl or cyclic amines, or wherein R⁷, and R⁸, together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O-and/or can be functionalized by one or multiple hydroxyl groups and/or mercapto groups, and wherein R⁷, R⁸, can be the same or different;
   wherein Xⁿ⁻ is an anion of an organic or inorganic acid, and
   wherein n ≥1, in particular 1, 2, or 3 , and
c) wherein the reaction takes place in a salt melt of the at least one ammonium salt of the general formulae (II).

It is understood that the ammonium salts of the general formulae (II) do not include such compounds where all of R⁷ and R⁸ are H; thus compounds such NH₄Cl or (NH₄)₂SO₄ are exempted. However, it may be of an advantage to add such unsubstituted ammonium salts to the reaction mixture as catalyst as described further below in more detail.

Thus, the present method is carried out in a salt melt of the at least one ammonium salt of the general formulae (II) at atmospheric pressure and does not require any longer applying pressure to the reaction mixture. Therefore the present method allows for the specific modification of a primary amino group of a suitable compound, such as a triazine compound, wherein a modification of the N-atom of the primary amino group occurs in a pressure free reaction environment.

This reaction can be described as a transamination reaction wherein a primary amino group of the triazine ring is replaced by the amino group of the ammonium salt of formulae (II). In the course of the transamination reaction the N atom of the ammonium salt of formulae (II) attacks the C-atom (being δ+) of the triazine ring adjacent to the exocyclic primary amino group. This provides a transition state in which the hydrogen atom of the amino compound of formulae (II) is transferred to the exocyclic primary amino group. The simultaneously occurring bond breakage provides the release of ammonia. In other words, the N-atom of formula (II) compound is now bond to the triazine ring and the primary amino group of the triazine is released in form of ammonia.

It is preferred if the R¹ and/or R² are a moiety of the formula R³-N-R⁴ or R⁵-N-R⁶ bound with its central nitrogen atom to the triazine ring of the structure of formula (I), wherein R³, R⁴, R⁵ and R⁶ mean independently from each other H, linear or branched C₁-C₁₀-alkyl (such as methyl, ethyl, propyl, butyl) or C₅-C₁₀-cyclo alkyl (such a cyclopentyl, cyclohexyl), wherein in each case one or multiple carbon atoms can be substituted by one or multiple oxygen atoms, wherein the oxygen atom has at least a distance of (-CH₂-)ₙ with n≥2, such as (-CH₂-CH₂-) from the N-atom, such as morpholine, ethyleneglycol or diethyleneglycol, or can be functionalised with hydroxyl or mercapto groups, such as hydroxyethyl, mercaptoethyl. In the latter case the N-atom is therefore substituted by an ether type moiety.

In a more preferred embodiment of the present method the triazine used in step a) is preferably of the general formulae (Ia) 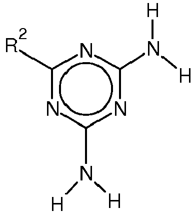 wherein R² has the above meaning. Thus, it may be preferred to use a triazine comprising at least two primary amino groups.

In the case of the compound of formulae (Ia)R¹ is a moiety of the formula R³-N-R⁴ bound with its central nitrogen atom to the triazine ring of the structure of formula (I), wherein R³ and R⁴ mean H.

In a most preferred embodiment of the present method melamine is used as triazine in step a). In this case R¹ and/or R² are a moiety of the formula R³-N-R⁴ or R⁵-N-R⁶ bound with its central nitrogen atom to the triazine ring of the structure of formula (I), wherein R³, R⁴, R⁵ and R⁶ mean H.

In a further embodiment of the present method the moieties R⁷ and R⁸ of the at least one ammonium salt of the general formulae (II) are selected from a group comprising linear or branched C₁-C₁₀-alkyl, C₅-C₁₀-cycloalkyl, or C₁-C₁₀-alkylsubstituted C₅-C₁₀-aryl or wherein R⁷ and R⁸ form together an C₅-C₁₀ alkyl ring, wherein in each case one or multiple carbon atoms can be substituted by one or multiple oxygen atoms, sulphur atoms and/or substituted nitrogen atoms and/or can be functionalized by one or multiple hydroxyl groups and/or mercapto groups. Preferred moieties R⁷ and R⁸ are selected from a group comprising methyl, ethyl, n-propyl, isopropyl, n-butyl, s-butyl, t-butyl, isobutyl, pentyl, cyclopentyl, hexyl, cyclohexyl, 2-ethyl-hexyl, octyl, decyl, stearyl, phenyl, toloyl, xyloyl, hydroxyethyl, hydroxypropyl.

It is furthermore preferred if the anion Xⁿ⁻ of the at least one ammonium salt of general formulae (II) is an anion selected from a group comprising halogenids, in particular chloride, bromide and fluoride, sulfate, hydrogensulfate, phosphate, hydrogendiphosphate, dihydrogenphosphate, nitrate, nitrite, tetrafluorborate, hexafluorphosphate, polyfluoralkansulfonate, bis(trifluormethylsulfonyl)imide, alkylsulfate, alkansulfonate, arylsulfonate, acetate.

In a most preferred embodiment of the present method the at least one ammonium salt of general formulae (II) is methylammoniumchloride, methylammoniumsulfate, dimethylammoniumchloride, dimethylammoniumsulfat, ethylammoniumchloride, diethylammoniumchloride, hydroxyl-ethylammoniumchloride and/or dihydroxy-ethylammoniumchloride.

In a variant of the present method the ratio of the at least one triazine of general formulae (I) and ammonium salt of general formulae (II) is at least 1 : 3, preferably at least 1 : 5, most preferably at least 1: 10. In general a higher amount of the ammonium salt increases the reaction velocity and the overall yield. Furthermore, an excess of the ammonium salt also shifts the alkylation to higher substituted triazines, like di- or trisubstitution on the triazine ring.

The present transamination reaction is preferably carried out at a temperature in a range between 120 and 280 °C, preferably between 150 and 230 °C, most preferably between 180 and 220°C. In general the conversion rate of the triazine increases with temperature, i.e. the higher the temperature the higher the yield of the transaminated product. This is most probably due to an increased amount of molten compounds in the reaction mixture.

It is also preferred, if the present reaction is carried out for at least 1 h up to 48 hours. The reaction time is preferably selected in dependency on the desired product ratio, in particular on the desired transamination rate. For example, if -in case of using melamine as starting compounds- the reaction is stopped after 4 hours or less than a high yield of a monoalkylated melamine is obtained. If the reaction time is over 5-10 h dialkylated melamine is primarily obtained. Therefore, depending on the reaction time and also of course the other reaction conditions as described it is possible to adjust the substitutional pattern of the triazine.

It is also desirable and preferred, if at least one ammonium salt of the general formulae (III) [NH₄⁺]ₙXⁿ⁻ with X and n having the above meanings is added to the reaction mixture. In this case the molar ratio of the at least one triazine of general formulae (I) and the ammonium salt of general formulae (III) can be at least 1: 0.1, preferably at least 1: 0.01. An increased catalyst amount has a positive influence on the reaction velocity and product composition. However, the compound of formulae (III) is still used in catalytic amounts and thus the compound of formulae (III) does not serve as reaction partner.

It is also preferred, if the transamination reaction is carried out in an inert gas atmosphere such as in the presence of helium, nitrogen, argon or in the presence of a gas which does not undergo any reaction at the described conditions such as ammonia. This reduces or even avoids side reactions.

It is also possible and preferred if after the reaction is completed the obtained product mixture is at first neutralised before any further work up steps are carried out. Neutralisation can be done by adding any suitable base such as NaOH or KOH. By doing so the amines and ammonia as well as a part of the side products and triazine are removed whereas the desired products are enriched in the solution.

The raw product, preferably obtained after neutralisation, undergoes further work up such as recrystallisation or extraction using as solvents water or alcohols, such as ethanol, butanol, isopropanol or mixtures thereof. The work up can be for instance carried out in such a way that the raw product is heated and dissolved in a solvent and the suspension is hot filtrated. The filtrate is subsequently cooled and the crystallized product is separated and analyzed.

The reaction mechanisms of two variants of the transamination are now exemplarily shown using melamine as triazine:

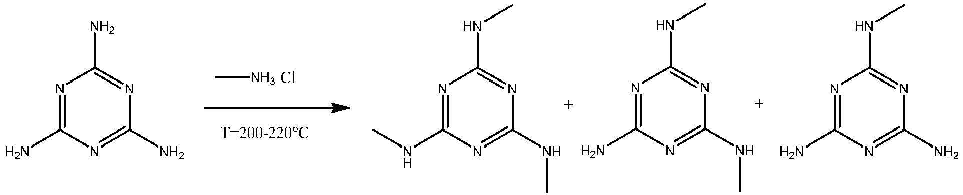

In above reaction scheme the three primary amino groups of melamine are substituted or undergo transamination in the presence of methylammoniumchlorid.

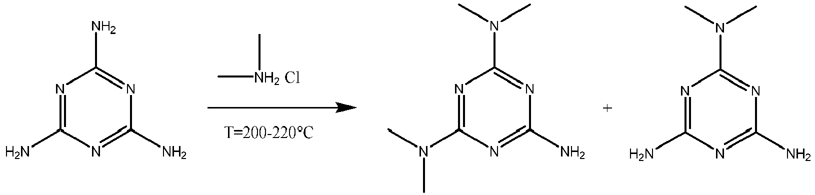

In above reaction scheme two primary amino groups of melamine are substituted or undergo transamination in the presence of dimethylammoniumchlorid. Depending on the reaction condition such as temperature, reaction time, amount of catalyst, ratio of reactants it is also possible that the reaction proceeds further under formation of N,N,N',N'-tetramethylmelamine (di-substitution) and/or N,N.dimethylaminomelamine.

It is to be understood that above reaction is only one possible example and that the present invention is not restricted to these specific compounds but that rather multiple different triazines and secondary amines may be used as described in detail above.

The compounds obtained by the present method are in particular useful as additives in polycondensation resins, like phenol-formaldeyhde resins or triazine-formaldehyde resins, such as melamine-formaldehyde-resins, melamine-urea-formaldehyde resins or urea-formaldehyde resins, for improving flexibility of said resins and for reducing brittleness of the resins and for postforming properties of the formed wood based panels.

In the context of the present invention the term "additives" means that the compounds can be added to the resins without undergoing any further reaction with the resin (for instance if the compound does not comprise any free primary amino group), or that the compounds can undergo a reaction with the resin (for instance if the compound still comprises a reactive hydrogen at any remaining amino group). In the latter case the additive can also be described as a type of comonomer.

Further details of the invention will be explained by the means of the following examples.

### Example 1:

Melamine (6.3 g, 50 mmol), methylammoniumchloride (10.1 g, 150 mmol) and ammoniumsulfate (0.66 g, 5mmol) are ground to a powder in a mortar. The reaction mixture is heated under argon for 24 hours to 200°C and is thereby mixed with a magnetic stirrer (a melt is obtained after about 1.5 hours which solidifies again after 10 hours). Samples are taken from said mixture (after 1 hour, 2 hours, 3 hours, 5 hours, 7 hours, 10 hours).

The reaction mixture is cooled to 100 °C after the end of the reaction and is mixed with water (50 ml). The mixture is neutralized at 80 °C with two normal NaOH (about 75 ml) whereby amines and ammonia are released. The reaction mixture is concentrated in vacuum to about half. Since after cooling no solid precipitates but an oily organic phase is separated the product mixture is extracted with ethyl acetate (3 x 50 ml). The unified organic phases are dried over Na₂SO₄ and is reduced in vacuum. A white solid is obtained: 4.3 g/ 56% calculated as N, N'-dimethylmelamine.

HPLC analysis of the product provides the following data: Melamine 0.2%; methylmelamine: 2.3%; N,N'-dimethylmelamine: 68.1%; trimethylmelamine: 29.3%.

The water phase was also reduced completely to dryness and the HPLC-analysis thereof provides the following data: Melamine 6.0%, methylmelamine 17.5%, N,N'-dimethylmelamine 74.3%, trimethylmelamine 2.1%.

The reaction is characterized by an initial induction time which results supposedly from the gradual melting of the reaction mixture. Melamine as well as methylammoniumchloride have a melting point which is above the reaction temperature of 200°C and thus the reaction is restricted at the beginning to the surface of the solid particles. Only when methylated melamine is formed the reaction mixture melts and a melt is obtained in which the starting materials dissolve at least partially. Only then, the reaction velocity reaches its maximum.

### Example 2:

Example 1 is repeated at 220°C under otherwise identical reaction conditions. After the end of the reaction the mixture is mixed at room temperature with 2N NaOH and is stirred overnight at room temperature. Thereby, a white solid precipitates. The suspension is placed in a refrigerator for 4 hours and is extracted by suction. The yield is 4.4 g/ 58% calculated as N,N' dimethylmelamine. HPLC analysis of the white solid provides the following composition: Methylmelamine: 1.3%, N,N' dimethylmelamine: 97.0%, N,N',N"-trimethylmelamine: 1.7%. The mother liquor is reduced in vacuum and has according to HPLC analysis the following composition: Melamine 1.1%; methylmelamine 7.7%, N,N' dimethylmelamine 44.2% and 47% N,N', N"-trimethylmelamine.

The reaction shows no initial induction time. The reaction under formation of N,N' dimethylmelamine occurs very rapidly. Already after 4 hours a yield of 60% of N,N' dimethylmelamine is obtained. The content increases afterwards only slowly. Also, the percentage of trimethylmelamine increases only slowly. The reason for that may be the use of only 3 equivalents methylammoniumchloride. This is exactly the amount which is required for complete formation of trimethylmelamine. However, the reaction mixture is depleted of methylammoniumchloride towards the end of the reaction what slows the velocity. Such a mixture ratio of melamine and methylammoniumchloride is of an advantage for the formation of N,N' dimethylmelamine. For the complete methylation of melamine to trimethylmelamine methylammoniumchloride has to be used in excess.

The conversion of melamine with methylammoniumchloride proceeds rather rapid and with almost no formation of side products. Due to different molar ratios of melamine and methylammoniumchloride the reaction can be guided to the formation of the two or three times methylated product. N.N' dimethylmelamine can be separated from the reaction mixture by crystallization in water with high purity. The extraction of the aqueous reaction mixture with ethylacetate can also serve for separating N,N' dimethylmelamine and trimethylmelamine. An enrichment of trimethylmelamine in the organic phase was noted while N,N' dimethylmelamine is present in both phases, whereby it can crystallize from the aqueous phase.

### Example 3:

Melamine (6.3 g, 50 mmol) and dimethylammoniumchloride (12.2 g, 150 mmol) are ground to a powder in a mortar. The reaction mixture is heated under argon for 24 hours at 200°C and is mixed with a magnetic stirrer. Samples are taken from the reaction mixture (after 1 h, 2h, 3h, 5h, 7h,10h).

After the end of the reaction the reaction mixture is cooled to 80°C and water (50 ml) is added. The mixture is subsequently cooled to room temperature and is mixed with 2N NaOH (75 ml). The obtained suspension is reduced in a rotary evaporator by about half, is placed into the refrigerator and is aspirated. The row yield is 6.8 g/ 88% (calculated as N,N-dimethylmelamine).

**Table 1**

| Reaction time (h) | Melamine (mass-%) | N,N-DMM (mass-%) | N,N,N',N'-TMM (mass-%) |
|---|---|---|---|
| 1 | 92 | 8 | 0 |
| 5 | 70 | 29 | 1 |
| 10 | 55 | 44 | 1 |
| 24 | 21 | 69 | 10 |

The product distribution depending on the reaction time is shown in Table 1.

The reaction time is too short for the formation of larger amounts of higher transamination products. The conversion of melamine with dimethylammoniumchloride is slower than the conversion with methylammoniumchlorid what might be due to the stronger sterical hindrance of the two times alkylated ammonium salt in respect to the monoalkylated ammonium salt of examples 1 and 2.

### Example 4:

A reaction of example 3 is repeated at 220°C under otherwise identical reaction conditions.

**Table 2**

| Reaction time (h) | Melamine (mass-%) | N,N-DMM (mass-%) | N,N,N',N'-TMM (mass-%) |
|---|---|---|---|
| 1 | 59 | 40 | 1 |
| 5 | 21 | 71 | 8 |
| 10 | 17 | 73 | 10 |
| 24 | 1 | 71 | 28 |

The product distribution depending on the reaction time is shown in Table 2.

The conversion of melamine to N,N-dimethylmelamine continues more rapidly than in example 3. The further conversion to N,N,N',N'-tetramethylmelamine is only very slow. This synthesis variant provides a good possibility for synthesizing N,N-dimethylmelamine after about 5-10 h. Higher transamination products are only obtainable after a longer reaction time at higher yield. The reaction time for synthesizing a larger amount of N,N,N',N'-tetramethylmelamine has to be at least 48 h.

### Example 5:

The synthesis procedure of example 3 is carried out at 200°C with dimethylammoniumsulfate instead of dimethylammoniumchloride. At the beginning, the reaction mixture forms a melt which can be stirred, however, after short time the reaction mixture is solidified (1-2 h). After 24 h, melamine is converted to 22 mass-% N,N-DMM and 8 mass-% N,N,N',N'-TMM.

Most important is the different reaction behavior compared to the Examples 1 to 4. In presense of dimethylammoniumsulfate the transalkylation of the second amino group of triazine starts immediately after the transalkylation of the first amino group.

## Claims

1. Method for modifying a triazine compound
comprising the steps of
a) Providing at least one triazine compound of the general formulae (I) 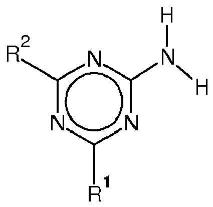 wherein
- R¹ and R² mean independently from each other Q¹ or a moiety of the formula R³-N-R⁴ or R⁵-N-R⁶ bound with its central nitrogen atom to the triazine ring of the structure of formula (I), whereat
- Q¹ means H, hydroxyl, a linear or branched C₁-C₃₀-alkyl or a cyclic substituent in form of a C₅-C₂₀-cycloalkyl, a C₅-C₂₀-aryl, a C₁-C₂₀-alkylsubstituted C₅-C₂₀-aryl, wherein in each case one or multiple carbon atoms can be substituted by one or multiple oxygen atoms, sulphur atoms, substituted nitrogen atoms and/or by one or multiple groups of the type -C(O)O-,-OC(O)-,-C(O)- and/or -OC(O)O-, and/or can be functionalized by one or multiple hydroxyl groups and/or mercapto groups,
- R³, R⁴, R⁵ and R⁶ mean independently from each other H, linear or branched C₁-C₂₀-alkyl, C₅-C₂₀-cyclo alkyl, C₅-C₂₀-aryl, C₁-C₂₀-alkylsubstituted C₅-C₂₀-aryl, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O)O-, and/or can be functionalized by one or multiple hydroxyl groups and/or mercapto groups; or an amide of a carboxylic acid or an imide of a cyclic dicarboxylic acid, and
b) Reacting said aminotriazine of the general formulae (I) with at least one ammonium salt of the general formulae (II)
[(R⁷R⁸H₂)N⁺]ₙXⁿ⁻
wherein R⁷ and R⁸ are linear or branched C₁-C₂₀-alkyl, C₅-C₂₀-cycloalkyl, or C₁-C₂₀-alkylsubstituted C₅-C₂₀-aryl or cyclic amines, or wherein R⁷ and R⁸ together form an alkyl ring, wherein in each case one or multiple carbon atoms can be substituted by one or multiple oxygen atoms, sulphur atoms and/or substituted nitrogen atoms and/or by one or multiple groups of the type -C(O)O-, -OC(O)-, -C(O)- and/or -OC(O) O-and/or can be functionalized by one or multiple hydroxyl groups and/or mercapto groups, and wherein R⁷ and R⁸ can be the same or different;
wherein Xⁿ⁻ is an anion of an organic or inorganic acid, and
wherein n ≥1, in particular 1, 2 or 3 , and
c) wherein the reaction takes place in a salt melt of the at least one ammonium salt of the general formuale (II).

2. Method according to claim 1, **characterized in that** the reaction is carried out at atmospheric pressure.

3. Method according to one of the preceding claims, **characterized in that** R⁷ and R⁸ of the at least one ammonium salt of the general formulae (II) are selected from a group comprising linear or branched C₁-C₁₀-alkyl, C₅-C₁₀-cycloalkyl, or C₁-C₁₀-alkylsubstituted C₅-C₁₀-aryl or wherein R⁷ and R⁸ form together an C₆-C₁₀ alkyl ring, wherein in each case one or multiple carbon atoms can be substituted by one or multiple oxygen atoms, sulphur atoms and/or substituted nitrogen atoms and/or can be functionalized by one or multiple hydroxyl groups and/or mercapto groups.

4. Method according to one of the preceding claims, **characterized in that** R⁷ and R⁸ are selected from a group comprising methyl, ethyl, n-propyl, isopropyl, n-butyl, s-butyl, t-butyl, isobutyl, pentyl, cyclopentyl, hexyl, cyclohexyl, 2-ethyl-hexyl, octyl, decyl, stearyl, phenyl, toloyl, xyloyl, hydroxyethyl, hydroxypropyl.

5. Method according to one of the preceding claims, **characterized in that** that Xⁿ⁻ of the at least one ammonium salt of general formulae (II) is an anion selected from a group comprising halogenids, in particular chloride, bromide and fluoride, sulfate, hydrogensulfate, phosphate, hydrogendiphosphate, dihydrogenphosphate, nitrate, nitrite, tetrafluorborate, hexafluorphosphate, polyfluoralkansulfonate, bis(trifluormethylsulfonyl)imide, alkylsulfate, alkansulfonate, arylsulfonate, acetate.

6. Method according to one of the preceding claims, **characterized in that** the at least one ammonium salt of general formulae (II) is methylammoniumchloride, methylammoniumsulfate, dimethylammoniumchloride, dimethylammoniumsulfate, ethylammoniumchloride, diethylammoniumchloride, hydroxyl-ethylammoniumchloride and/or dihydroxyethylammoniumchloride.

7. Method according to one of the preceding claims **characterized in that** the molar ratio of the at least one triazine of general formulae (I) and the ammonium salt of general formulae (II) is at least 1:3, preferably at least 1:5, most preferably at least 1:10.

8. Method according to one of the preceding claims, **characterized in that** at least one ammonium salt of the general formulae (III) [NH₄⁺]ₙXⁿ⁻ with X and n having the above meanings is added to the reaction mixture.

9. Method according to claim 8, **characterized in that** the molar ratio of the at least one triazine of general formulae (I) and the ammonium salt of general formulae (III) is at least 1: 0.1, preferably at least 1: 0.01.

10. Method according to one of the preceding claims, **characterized in that** the reaction is carried out at a temperature in a range between 120 and 280 °C, preferably between 150 and 230 °C, most preferably between 180 and 220 °C.

11. Method according to one of the preceding claims, **characterized in that** the reaction is carried out for at least 1 h up to 48 hours.

12. Method according to one of the preceding claims, **characterized in that** the triazine used in step a) is of the general formulae (Ia) 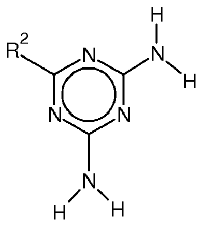 wherein R² has the above meaning.

13. Method according to one of the preceding claims, **characterized in that** melamine is used as triazine in step a).
